# EUROPEAN PATENT APPLICATION

(11) **EP 2 272 510 A1**
(43) Date of publication of application: **12.01.2011**
(21) Application number: 09290541.3
(22) Date of filing: 06.07.2009
(51) Int. Cl.: A61K 31/19, A61K 31/197, A61K 31/201, A61P 33/02

(54) **Inhibitors of the proline racemase of trypanosoma cruzi**

(71) Applicant: Institut Pasteur, 75015 Paris (FR)
(72) Inventor: Minoprio, paola, 94350 Villiers sur Marne (FR); Berneman, Armand, 75011 Paris (FR); Blondel, Arnaud, 75015 Paris (FR)
(74) Representative: Thomas, Dean

(57) **Abstract**

The present invention relates to a new class of compounds of the general Formula (i): 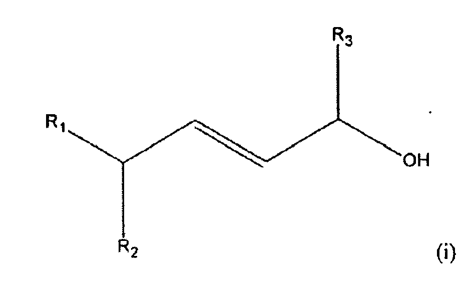 wherein R₁ is selected from the group consisting of: a substituted or unsubstituted C₁-C₁₀ alkyl group and a polar atom selected from the group consisting of: oxygen, nitrogen, sulfur or halogen atom; R₂ and R₃ are polar groups chosen from the group consisting of: =O, =NH, -NH₂, -OH, =S, -X₁ wherein X₁ is a halogen atom; and isomers thereof, which can in particular be used as inhibitors of Proline Racemase, as a medicament and as a component of a pharmaceutical composition.
Also disclosed are *in silico* methods to determine inhibitors of a target using a novel structural progression approach.

## Description

The present invention concerns novel inhibitors of proline racemase an essential enzyme of several important organisms. In particular the present invention relates to a novel class of inhibitor of the proline racemase enzyme of *Trypanosoma cruzi* consisting of Formula (i). The present invention also relates to novel methodologies to identify inhibitors of this important class of enzyme and in particular relates to *in silico* methods to model ligand integration and select suitable candidate inhibitors.

Chagas' disease is one of the main causes of death due to amongst other causes heart failure in Latin American countries. Chagas' disease is caused by the obligate parasite *Trypansoma cruzi.* The disease is most commonly transmitted by an insect vector which has taken a blood meal from a parasite infected individual, but can also be transmitted via a blood transfusion or organ/tissue transplant.

It is the third largest health burden in Latin America where its impact is largely limited only by vector transmission frequency [1]. 10-12 million people are currently infected and around 100 million people are at risk of catching this disease. Approximately 10% of the affected adult population will die from acute disease which is incurable and fatal to children fatal to children younger than 2 years old. No vaccines are available and current therapies are effective in the acute phase of the disease and their success in the chronic phase remains a matter of debate.

Outbreaks in Latin American countries occur regularly and an outbreak in French Guyana in 2004 [2], resulted in the issuance of a Commission Directive from the European Parliament to exclude from blood banks all donors that have lived for more than three months in countries where Chagas' disease is endemic and in particular this directive implemented eligibility criteria for donors of blood, blood components, cells and tissues [3]. Recent increases due to congenital transmission, blood transfusion and transplantation has further focused the attention of Public Health authorities in both Europe and the USA upon the dangers to public health presented by Chagas' disease [4, 5, 6].

Antiparasitic treatment is most effective early in the course of infection and presently two drugs are available to treat infected individuals and specifically to kill parasites in these individuals: Nifurtimox (3-methyl-4-5'-nitrofurfurylidene-amino tetra hydro 4H- 1,4-thiazine-1,1-dioxide) and Benznidazole, a nitroimidazole derivative (N- benzyl-2-nitro-1-1-imidazole acetamide) [7]. Nifurtimox use has mostly been discontinued and so Benznidazole is today the only nitro-bearing chemotherapeutic agent prescribed for the treatment of patients [8].

Although the mechanism of action for Benznidazole has not been fully elucidated, the drug appears to interfere with the redox cycles of intracellular and extracellular parasite forms and partially reduces metabolites [9,10]. Several toxic but reversible side effects of this drug have been described such as general allergic dermopathy, peripheral polyneuropathy, leucopeny, anorexy, convulsions, etc [8]. Major complications include agranulocytosis and graulocytopenic. Benznidazole is therefore unsatisfactory due to its toxicity and low efficacy in treating the chronic phases of the disease, although most of cases are discovered at that stage [11,12].

Resistance to these drugs has been reported [31]. Moreover, a 10-year study of chronic administration of antiparasitic drugs in Brazil has revealed that current drugo treatment regimens do not fully remove parasites from the blood of infected individuals [32].

A complementary strategy would be to eradicate/suppress the insect vector populations and although this may address part of the incidence of this disease in humans, new more efficient treatments are also needed and must be considered a priority [13,14].

The inventors have previously identified and characterised the secreted and intracellular forms of *Trypanosoma cruzi* proline racemases (*Tc*PRACs), enzymes that can be detected at all life cycle stages of the parasite [15,16]. For instance, the secreted form of the enzyme is a B-cell mitogen [16,17] that contributes to parasite escape and persistence in the host.

*Tc*PRAC catalyses the interconversion of L- and D- proline enantiomers. This group of enzymes has not been found in many organisms in which searches have been made and in particular, functional PRAC enzymes have only been found in *Trypanosoma* spp and *Clostridium* spp. to date.

*Tc*PRAC is a promising lead in the development of new therapies against Chagas' disease since parasites whose PRAC genes have been genetically knocked out are not viable, whereas overexpressors of PRACs are more virulent to host cells [16]. Moreover, a clear dose-dependent effect has previously been shown where increasing concentrations of pyrrole carboxylic acid (PYC), a competitive (but non-soluble) inhibitor of PRAC [18].

Seeing these problems with the present treatments for the disease causing agent of Chagas' disease the inventors have now found a new class of compounds of the general Formula (i): 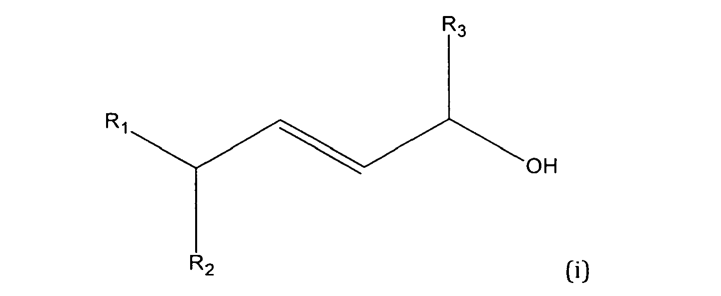 wherein R₁ is selected from the group comprising: a substituted or unsubstituted C₁-C₁₀ alkyl group and a polar atom selected from the group comprising oxygen, nitrogen, sulfur or halogen atom; R₂ and R₃ are polar groups chosen from the group comprising of: =O, =NH, -NH2, -OH, =S, -X₁ wherein X₁ is a halogen atom; and isomers thereof, which can be used to inhibit the PRAC enzyme and in particular can be used as a medicament.

In the present Patent Application a C₁-C₁₀ alkyl group is used to refer to monovalent aliphatic hydrocarbon radicals which comprise between one and ten carbon atoms and which may be linear, branched and also may optionally have been substituted with a halogen atom, preferably a bromine atom.

The inventors have used a number of approaches to identify new inhibitors of *Tc*PRAC. They firstly used a classical Medicinal Chemistry approach to improve the solubility and affinity of the known inhibitor PYC. Several PYC analogues were synthesized (imidazoles, pyrazoles, pyridines and oxazoles), and two of these soluble PYC-derivatives, containing respectively a Chloro or a Bromo at C-4, induced weak inhibitions of *Tc*PRAC activity (see Table1 below for further details of the compounds tested).

In a second complementary approach using available *Tc*PRAC functional and biophysical data, the inventors used complementary molecular simulation studies to create structural models suitable to the screen chemical libraries. This additional *in silico* data in combination with data derived from crystallography and ITC calorimetry allowed the inventors to generate a number of conformational models compatible with the transition states between the active and the inactive forms of the enzyme, that is the ligand bound and non-ligand bound forms. From this the inventors were able to design a new class of *Tc*PRAC inhibitors of which they synthesised and tested two examples which had superior inhibitory properties to PYC.

In particular alkyl group is selected from the group consisting of: methyl, ethyl, propyl, butyl, pentyl.

In particular alkyl group is substituted with a halogen atom.

In particular the halogen atom is selected from the group consisting of: fluorine, chlorine, bromine, iodine.

In particular the compound is (*E)*-4-oxopent-2-enoic acid (Formula (ii)) or a derivative thereof.

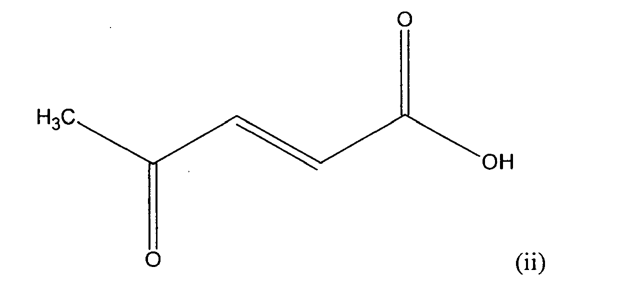

In particular the compound is (*E)*-5-bromo-4-oxopent-2-enoic acid (Formula (iii)).

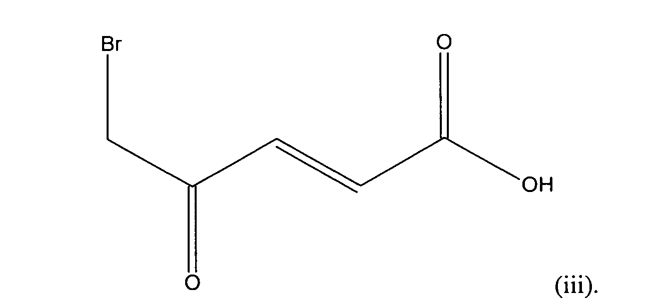

The inventors have designed, synthesized and tested against live *Trypanosma cruzi* parasites two compounds *(E)*-4-oxopent-2-enoic acid (X-beige or OxoPA, C₅H₆O₃, MW 114,10, Formula (ii)) and its derivative *(E)*-5-bromo-4-oxopent-2-enoic acid (22-ocre or Br-OxoPA, C₅H_{S}O₃Br, MW 193,1, Formula (iii)). These compounds are 2 to 3 times better inhibitors of *Tc*PRAC than PYC. OxoPA and Br-OxoPA molecules diluted in DMSO induce superior decreases of mammalian host cell infection and parasite fate in comparison to PYC. These data consolidate these molecules as potential new therapeutics against diseases where active proline racemase plays an essential role such as Chagas' disease as well as other important diseases such as *Trypanosoma vivax* a major parasite of cattle in Africa and South America as well as *Clostridium difficile* the bacteria most implicated in antibiotic resistant infections in hospitals and clinics as well as a serious pathogen in diseases of the digestive tract.

According to a further aspect of the present invention there is provided a pharmaceutical composition comprising at least one compound according to the present invention and a pharmaceutically acceptable carrier.

In particular the compound is in the form of a pharmaceutically acceptable salt or other pharmaceutically acceptable derivative of said compound.

Pharmaceutical salts typically are more soluble and more rapidly soluble in stomach and intestinal juices than non-ionic species and so are useful in solid dosage forms. Furthermore, because their solubility often is a function of pH, selective dissolution in one or another part of the digestive tract is possible and this capability can be manipulated as one aspect of delayed and sustained release behavior. Also, because the salt-forming molecule can be in equilibrium with a neutral form, passage through biological membranes can be adjusted. Formulations for administration intravenously, intramuscularly, subdermally or via other known administration routes are also encompassed by the present invention.

Specific methods to generate pharmaceutically acceptable salts of compounds are well known in the art [40, 41] and such methods are incorporated in the present Patent Application.

According to a further aspect of the present invention there is provided the use of a compound according to the present invention as a medicament.

There is also provided the use of a compound according to the present invention for the preparation of a medicament to treat *Trypanosoma cruzi, Trypanosoma vivax* or *Clostridium difficile* infection. More generally the present invention provides the use of a compound according to the present invention for the treatment of a disease in which the agent of the disease comprises an essential PRAC enzyme.

The present invention also relates to *in silico* methods to model the interactions of an enzyme such as PRAC with its ligand so as to identify novel ligands for use *in vivo* or *in vitro* for amongst other purposes as research tools, therapeutics, diagnostic reagents and components of further reagents or compositions for any of these purposes.

In particular this *in silico* simulation is a computational stimulation of an enzyme as it changes between two states. The method consists of modelling the conformational changes of the enzyme *in toto* or a portion thereof as it changes from a first state to a second state through a specific set of intermediary structures chosen by the simulation from a number of possible intermediary structures. In particular the first state is the enzyme unbound to its ligand and the second state is the enzyme bound to its ligand. The passage of the enzyme through its intermediary structures being modelled based upon a number of parameters and calculated using a number of methods as detailed herein.

For a better understanding of the invention and to show how the same may be carried into effect, there will now be shown by way of example only, specific embodiments, methods and processes according to the present invention with reference to the accompanying drawings in which:

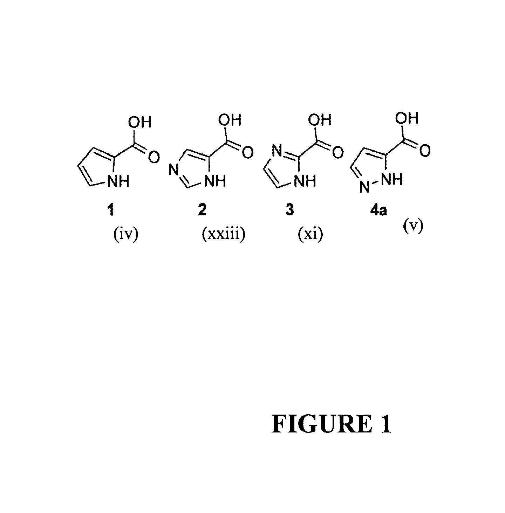

Figure 1. shows the structure of initial compounds and analogues, in which 1 is PYC (Sigma , P7 360-9), 2 (Interchim, custom synthesized) and 3 (Maybridge, CC0890 1) are Nitrogen-bearing analogues which were found to be inactive with PRAC and 4a (see methods below for synthesis method) is a Nitrogen-bearing analogue made by a potassium permanganate oxidation of 3-methylpyrazole [22,23] which is very weakly active with PRAC.

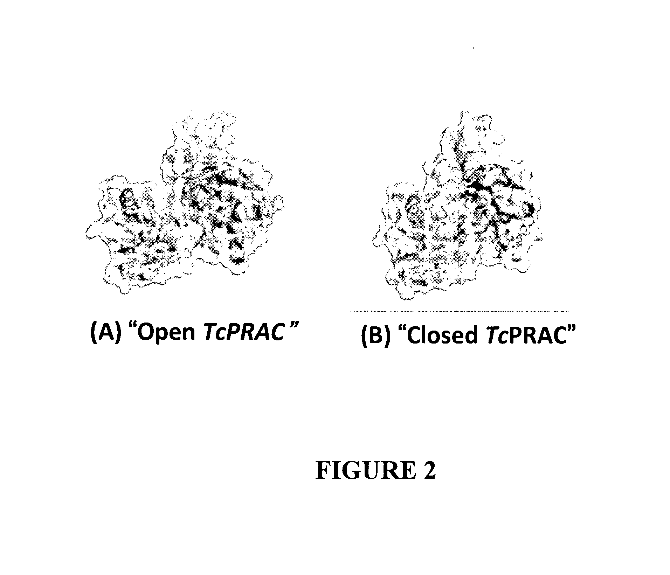

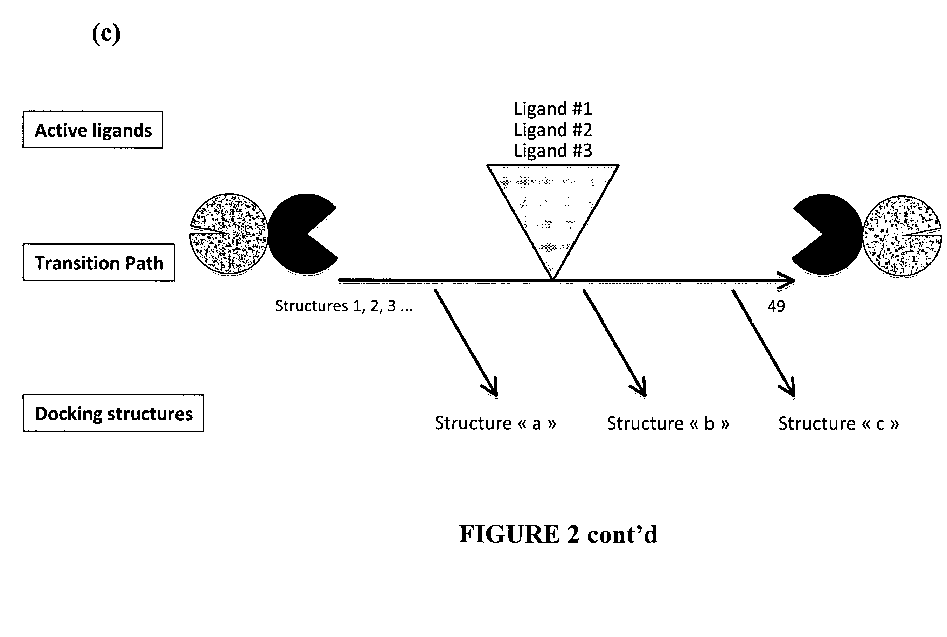

Figure 2. shows structural rearrangement of TcPRAC (A) In the absence of PYC, the monomer displays a more relaxed, open structure with the active site accessible from the bulk solvent (the inhibitor is shown here for visualization purposes). (B) Closed structure of the *Tc*PRACA monomer in the presence of PYC, with the substrate-binding cavity completely buried within the monomer. [20] (C) shows a schematic representation of the modeling of the transition of the PRAC enzyme following its recognition and binding of a ligand.

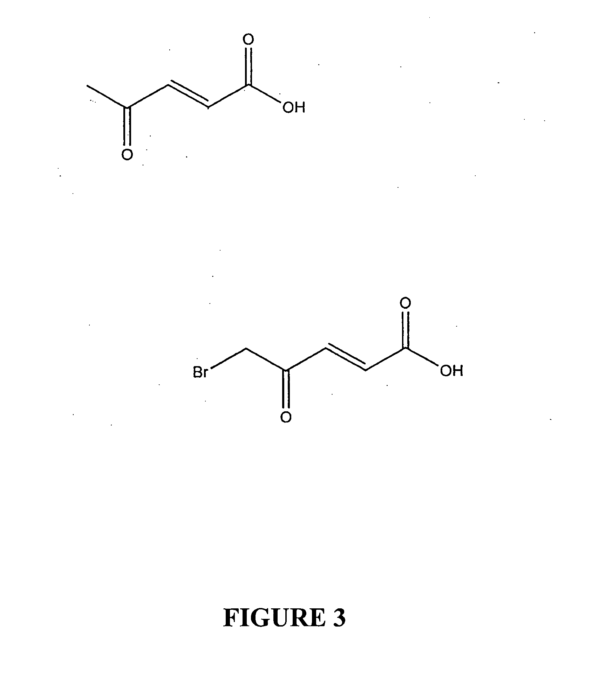

Figure 3. shows the structures of *(E)*-4-oxopent-2-enoic acid (OxoPA, C_{S}H₆O₃, MW 114,10) and its derivative *(E)*-5-bromo-4-oxopent-2-enoic acid (Br-OxoPA, C₅H₅O₃Br, MW 193,1).

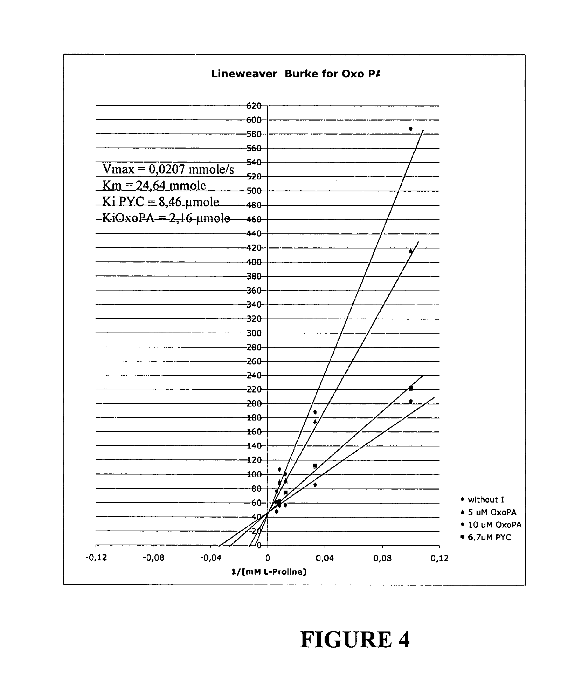

Figure 4. Concentrations of L- and D-proline were determined polarimetrically from the optical rotation of the solution at 365 nm in a cell of 10 cm-path length, thermostated at 37°C. Assays were done with 20 µg / ml of recombinant protein, 40 mM L-proline in 0.2 M sodium acetate, pH 6.0, in a final volume of 1.5 ml. Optical rotation was measured every 5 s during 10 min. Double reciprocal plot kinetics of 22,4 µg / ml *Tc*PRAC in the presence of 6,7 µM PYC competitive inhibitor (squares), 5 µM OxoPA (triangles) and 10 µM OxoPA (circles) or buffer (lozenges) in function of proline concentration, where 1/V (V, initial speed, mol.sec-1) is plotted in function of 1/S (S, substrate concentration, M). Graphic representation and linear curve regression allowed the determination of Kᵢ as [I]/ apparent Kₘ / Kₘ)-1). Graphic representation and Lineweaver-Burk linear curve regression allowed the determination of Kᵢ as [I] / [(apparent Kₘ/Kₘ) - 1]. All reagents were purchased from Sigma.

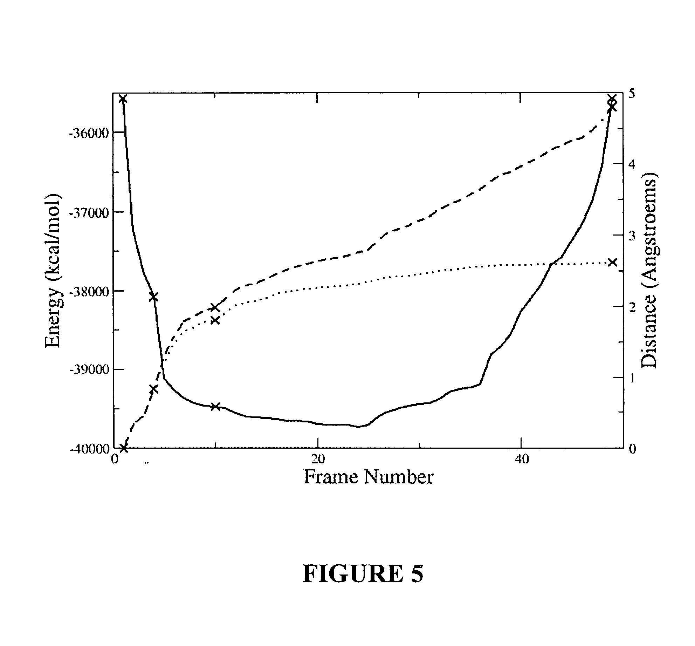

Figure 5: Energetic profile and metric quantities for the transition path of TcPRAC. The energy profile (full line) show that intermediate states have low energy and present no energy barriers. Dotted and dashed lines show the distance from first structure and the cumulative distance from first structure covered respectively. Little swerving is necessary to avoid energy barriers. The points corresponding to the intermediate structures used for the screening are marked by crosses.

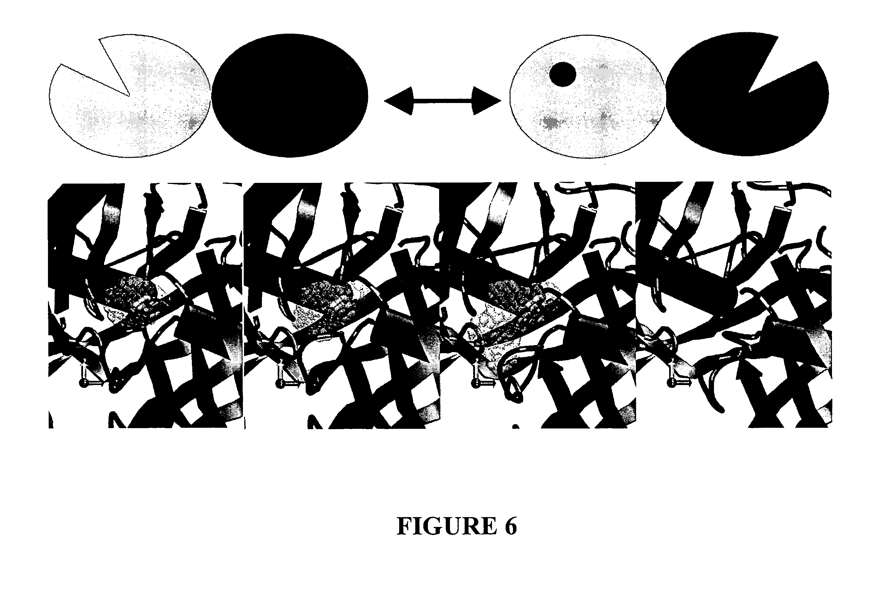

Figure 6: Reaction path. a) Schematic view of path building. A symmetric form of the crystallographic structure was built and a series of structures bridging the two forms without steric collision was calculated. Protomers are respectively in light and dark grey and PYC in black. b) Four of the 49 structures defining the path were selected for the virtual screening. Secondary structures of the protein are shown schematically with the same colors. Transparent spheres show the void volume in the pocket. The ligand is inside the first three structures.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example 1. Material and Methods

### 1.1 Synthesis of PYC derivatives

The following compounds and intermediate derivatives: 1H-pyrazole-3-carboxylic acid, 4-chloro-1H-pyrazole-3-carboxylic acid, 4-bromo-1H-pyrazole-3-carboxylic acid, 5-methyl-1H-pyrazole-3-carboxylic acid, 4-chloro-5-methyl-1H-pyrazole-3-carboxylic acid, 4-benzyl-1H-pyrazole-3-carboxylic acid ethyl ester, 3-benzyl-4-hydroxy-5-oxo-2-phenethyl-2,5-dihydrofuran-2-carboxylic acid ethyl ester, 4-(carboxymethyl)-1H-pyrazole-3-carboxylic acid dimethyl ester, 4-methyl-1H-pyrazole-3-carboxylic acid ethyl ester, 4-(trifluoromethyl)-1H-pyrazole-3-carboxylic acid ethyl ester, 4-ethyl-1H-pyrazole-3-carboxylic acid ethyl ester, 4-propyl-1H-pyrazole-3-carboxylic acid ethyl ester, 4-isopropyl-1H-pyrazole-3-carboxylic acid methyl ester, 4-isobutyl-1H-pyrazole-3-carboxylic acid methyl ester, 4-pentyl-1H-pyrazole-3-carboxylic acid ethyl ester, 4-phenyl-1H-pyrazole-3-carboxylic acid methyl ester, 4-(pyridin-2-yl)-1H-pyrazole-3-carboxylic acid ethyl ester, 4-(pyridin-3-yl)-1H-pyrazole-3-carboxylic acid ethyl ester, 4-(pyridin-4-yl)-1H-pyrazole-3-carboxylic acid ethyl ester, 4-methyl-1H-pyrazole-3-carboxylic acid, 4-(trifluoromethyl)-1H-pyrazole-3-carboxylic acid, 4-ethyl-1H-pyrazole-3-carboxylic acid, 4-propyl-1H-pyrazole-3-carboxylic acid, 4-isopropyl-1H-pyrazole-3-carboxylic acid, 4-isobutyl-1H-pyrazole-3-carboxylic acid, 4-pentyl-1H-pyrazole-3-carboxylic acid, 4-phenyl-1H-pyrazole-3-carboxylic acid, 4-benzyl-1H-pyrazole-3-carboxylic acid, 4-(pyridin-2-yl)-1H-pyrazole-3-carboxylic acid, 4-(pyridin-3-yl)-1H-pyrazole-3-carboxylic acid, 4-(pyridin-4-yl)-1H-pyrazole-3-carboxylic acid, 4-(carboxymethyl)-1H-pyrazole-3-carboxylic acid; were synthesized by one or more of the following there methods:
**Method A:** Compounds **4a-e** (Formula xxiii) were obtained via the controlled oxidation of 3-methylpyrazoles derivatives **5a-e** (Formula xxii) using potassium permanganate. This method was found to be quite limited by the extensive decarboxylation observed in some cases in refluxing water whereas the lack of solubility of the reaction substrates at room temperature often prevented less stringent methyl oxidation. This method is shown in scheme 1 below: 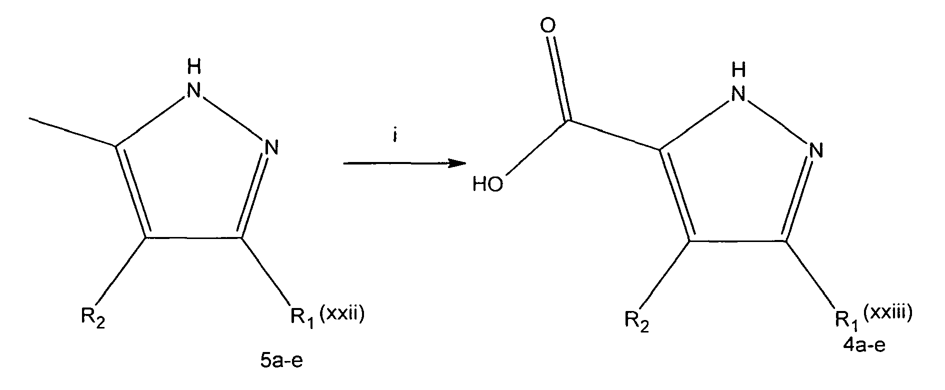 a: R₁, R₂ = H, H // b: R₁, R₂ = H, Cl // c: R₁, R₂ = H, Br // d: R₁, R₂ = Me, H // e: R₁, R₂ = Me, Cl.
   **Scheme 1: i**: KMnO₄, H₂O, either at 25°C or under refluxing water.
**Method B** is based on the recently reported preparation of ethyl-3-pyrazolecarboxylate from ethylpyruvate [42]. Using the reaction between dimethylformamide dimethylacetal and two pyruvate derivatives, followed by the addition of the hydrazine on the resulting Mannich base, the inventors thus extended this original method. The quite low yields obtained were due to the occurrence of many side compounds.
   In the first instance, compound **7** (Formula xxv) was isolated in 18 % yield and fully characterized. It results from a dimerization of the benzyl ethylpyruvate derivative **6a** (Formula xxiv). A related preparation of such compound has actually been reported [43]. From esters **8a** and **8b** (Formula xxvi), an acidic hydrolysis led anyway to sizable amount of the target analogues **9a** and **9b** (Formula xxvii). Method B is shown in scheme 2 below: 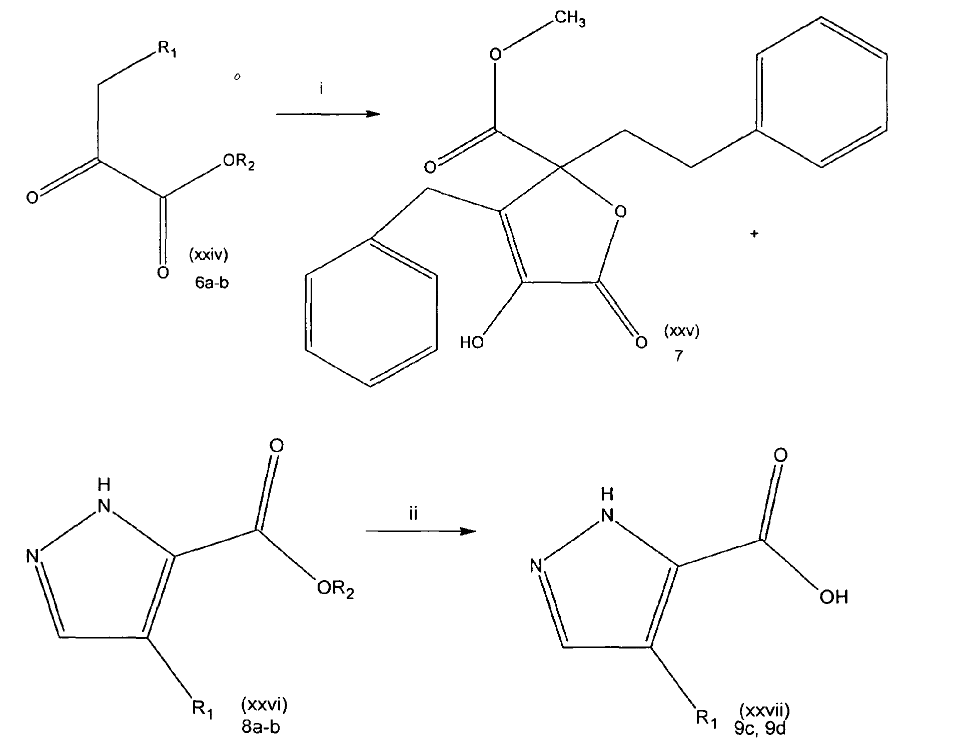 a: R₁, R₂ = CH₂C₆H₅, Et // b: R₁, R₂ = CH₂CO₂Me, Me // c: R₁= CH₂C₆H₅ // d: R₁ = CH₂CO₂H
   **Scheme 2: i:** a) DMF dimethylacetal. b) NH₂NH₂, HCl. **ii:** H₃OCl, reflux.
**Method C** use of the previously described cycloaddition of diazomethane on acrylate esters **10a-k** (Formula xxviii) which provides the intermediate **11a-k** (Formula xxix) in solution and is followed by an oxidative rearrangement into 4-substituted 3-pyrazole esters **12a-k** (Formula xxx) by using bromine [44, 45].As described in the experimental part, the unavailable α,β-unsaturated esters were prepared by esterification the commercially available acid or using the greatly optimized synthetic method based on the condensation of malonic acid monoethylester on aldehydes (List, 2006). The use of pure diazomethane was avoided in order to minimize the handling of this carcinogenic as well as explosive substance. Instead, we used a comparatively safer one pot protocol from N-methylnitrosourea [46] with only a phase separation step using a freezing method. This was followed by an acidic hydrolysis of the resulting ester function and provided compounds **13a-k** (Formula xxxi). This method is shown in scheme 3 below: 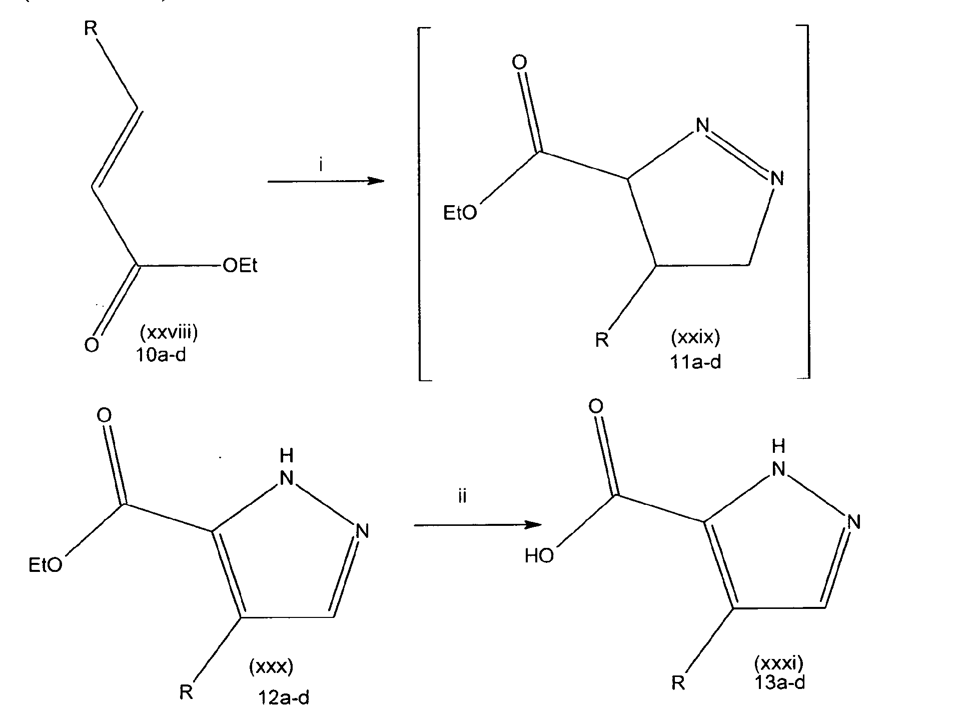 a: R=CH₃//b: R = CF₃ // c: R = Et // d: R = Pr // e: R = iPr // f: R = CH₂iPr // f: R = Pent // h: R = Bn // i: R = 2-py // j: R = 3-py // k: R = 4-py
   **Scheme 3:** i: CH₂N₂, Et₂O. **ii:** H₃OCl, reflux.

### 1.2 Molecular dynamics and models for docking:

A framework approach was used to calculate plausible low energy and connected intermediate states in the *Tc*PRAC conformational transitions. The catalytic site of *Tc*PRAC is small, approximately the volume of a proline amino acid and previous virtual screenings have previously been performed with designed pharmacophores (data not shown) yielded small and metabolite like molecules.

The inventors built possible intermediates states in the catalytic mechanism which involves the closing/opening of the *Tc*PRAC catalytic pocket, that is between its unliganded/opened and liganded/closed forms) [20]. This allowed the inventors to widen the search to larger and chemically more diverse candidate inhibitors. Specific criteria were chosen to establish the number of intermediate structures sufficient to describe the transition, the maximum energy along the path and the curvilinear length sufficient to complete the transition.

The path refinement was based on the description of conformational transition by a series of intermediate structures which are all below an energetic threshold and for which all linear interpolation between any pairs of consecutive structures are also below that threshold. The improvement of the path is the result of general refinement and identification of short-cuts or by-passes.

Briefly, the method included the following procedures which can be combined: a so called "backward" reduction method, a so called search for wider by-passes, an Heuristic search for by-passes: systematic search, an Heuristic to search for by-passes: priority search, criteria to select by-passes and/or a tree search to assemble by-passes (all at once or by sub-sections) (see Figure 2).

Four conformational models were chosen to perform the virtual screening of several thousand molecules out of two accessible chemical libraries: ChemDiv, Inc (San Diego, California, USA) and Chimiothèque Nationale (CNRS, France). The following programs were used: FlexX 2.0.3 [47]. Atomic coordinates and structure factors of *Tc*PRAC can be found in Protein Data Bank, www.pdb.org (PDB ID codes 1W61 and 1 W62).

### 1.3 Preparation of recombinant TcPRAC :

Recombinant *Trypanosoma cruzi* Proline racemase (SEQ ID NO: 1) was produced in *E. Coli* BL21 (DE3) (Invitrogen) and purified with immobilized metal affinity chromatography on nickel columns, as previously described [24].

### 1.4 Racemization assays of L-Proline and evaluation of inhibitors:

Optimum Proline racemization conditions for *Tc*PRAC were determined using 10-20 mM L-Proline in 0.2 M NaOAc, as a function of pH, as described [24].

Briefly, classical assays of Proline racemization induced by *Tc*PRAC were performed in tubes in a final volume of 1.5 ml and optical rotations measured in a polarimeter 241MC (Perkin Elmer). Parallel assays of Proline racemization were transposed into microplates in a final volume of 100 µl, as follows: serial dilutions of L- Proline ranging from 40 mM to 2.5 mM were prepared in 0.2 M sodium acetate pH 6.0 containing 0.25 mM r*Tc*PRAC and loaded into microplate wells in the presence or in the absence of 0 - 20 µM pyrrole-2 carboxylic acid (PYC) or of similar dilutions of unknown potential inhibitors. Specific control wells were included omitting the *Tc*PRAC and/or the PYC.

Microplates were incubated for 30 minutes at 37° C. Enzyme activity was then inactivated by heating the plate for 15 seconds in a microwave oven at 900 watts after shifting the pH of wells to 8.3 by addition of 6.8 µl of 0.235 M sodium Pyrophosphate. 50 µl of each well were used to test the presence of D-Proline using the DAAOx (D-amino acid oxidase) test described below [25]. L- amino acids may react at very low rate with DAAOx. For instance, DAAOx reacts with L-Proline 300 times slower than D-Proline. Consequently, when assessing amino acid racemization products, standards should consist of serial dilution of equimolar mixtures of specific D- and L-amino acids ranging from 0 to 20 mM (10 mM final) to take into account any interference of possible reactivity with DAAOx of the L- form of amino acids present in the samples.

### 1.5 D- amino acid oxidase microtiter plate test:

Solution A consisting of 0.2 M sodium acetate/0.016 M sodium pyrophosphate (Pop) buffer, pH 8.3 was used to prepare the enzyme stock solutions conserved in 50% glycerol, namely: 50 U/ml D-amino acid oxidase from pig kidney (DAAOx, Sigma) and 5000 U/ml Horse Raddish Peroxydase (HRP, Sigma). Stock solutions of L- proline (1M), D- proline (1M) and Pyrrole-2-carboxylic acid (10 mM ) (PYC, Sigma) are also prepared in solution A. 10⁻¹ M FAD (Sigma) stock was prepared in water. Solution B consists in adding to solution A the following reagents at a final concentration in the assay of 1 U/ml for DAAOx, 0.5 mg/ml orthophenylenediamine (OPD, Sigma), 37.5 U/ml HRP and FAD (1.5x10⁻⁵ M FAD). The test is run in 96 wells micro plates (Nunc, Denmark). In the first step reaction, 50 µl of each experimental or standard samples diluted in solution A are loaded per well previously containing 50 µl of solution B. Plates are incubated for 1 hour at 37° C in the dark. The reaction is stopped by addition of 50 µl of 3 N HCl to the wells. OD 490nm - 650nm is obtained with a microtiter plate reader (Molecular Devices), and compared to those obtained with blank control wells.

### 1.6 Parasites:

Cell culture-derived trypomastigotes from *T. cruzi* CL Brener (clone F11-F5) were isolated from the supernatant of Vero cells, which had been previously infected with bloodstream trypomastigotes [26].

### 1.7 Cell cultures:

Vero cells (from green monkey kidney) were seeded at a density of 15x10⁶ cells per 25cm² culture flasks or at 5 x10⁴ cells/well into LabTek slides. The cultures were maintained in RPMI 1640 medium supplemented with 5% fetal bovine serum and kept at 37°C in an atmosphere of 5% CO₂. Cell culture-derived trypomastigotes from *T.cruzi* CL Brener (clone F11-F5) were isolated from the supernatant of Vero cells, which had been previously infected with bloodstream trypomastigotes. Vero cell cultures were infected for 17h at 37°C with cultured trypomastigotes at a 10:1 parasite/cell ratio in the presence or in the absence of increasing doses of freshly made dilutions (3-30 µM) of *(E)*-4-oxopent-2-enoic acid (OxoPA) and its derivative *(E)*-5-bromo-4-oxopent-2-enoic acid (Br-OxoPA) [21], previously dissolved in dimethyl sulfoxide (DMSO). For comparison, cultures were seeded with or without 10-1000 µM specific proline racemase inhibitor pyrrole-2-carboxylic acid (PYC) also diluted in DMSO. To verify if proline racemase inhibitors could also exert an effect on the parasite intracellular cycle after 17 hours of parasite-host cell interaction, all the wells were then rinsed three times to eliminate all extracellular parasites and new medium was added to the cultures that were incubated for up to 48 hours with fresh dilutions of the inhibitor compounds. After the different treatments, cultures were washed with PBS, fixed and stained with Giemsa. The numbers of infected host cells and of parasites *per* infected cell were then scored in at least 400 host cells in independent experiments, run in duplicate. Results are expressed as the percentage of infected cells, the mean number of parasites per infected cell or by the endocytic index (EI) which represents the percentage of infected cells multiplied by the mean number of parasites per infected cell [27]. As controls, parasites were incubated only in culture medium, or in medium with comparable DMSO concentrations.

### 2. Results

### 2.1 Synthesis and analysis of analogues of PYC reveals two new inhibitors of proline racemase.

The inventors began by testing some commercially available variants of pyrrole carboxylic acid PYC for inhibitory effects upon *Tc*PRAC. The compounds are shown in Figure 1 and were obtained respectively from the commercial suppliers Interchim or Maybridge or the synthesis method detailed above. The effects of the compounds uponTcPRAC activity were assayed as detailed above. None of the tested compounds showed comparable inhibitory effects upon *Tc*PRAC to PYC.

From these results (Figure 1), a range of other 3-carboxylpyrazoles derivatives bearing substituents on carbon 4 and 5 were prepared..

Using classical Medicinal Chemistry approaches the inventors then sought to improve the solubility of the competitive (but non soluble) inhibitor of proline racemases PYC, as well as its affinity for the *Tc*PRAC catalytic pocket (6 microM) [15].

Primarily, several PYC analogues were synthesized (i.g. imidazoles, pyrazoles, pyridines and oxazoles) (see Material and Methods) [22] [28]. Therefore, while presenting better solubility those compounds did not present better affinity to *Tc*PRAC than that observed with PYC. Only two of those soluble PYC-derivatives, namely 4-chloro-1H-pyrazole-3-carboxylic acid and 4-bromo-1H-pyrazole-3-carboxylic acid, containing respectively a Chloro or a Bromo at C-4, induced weak inhibitions of *Tc*PRAC activity while the other compounds induced faint or no activities (Table 1).

**Table 1. PYC analogues**

| Name | Structure | Solubility | *Tc*PRAC Inhibition* |
|---|---|---|---|
| Pyrrole 2-carboxylic | 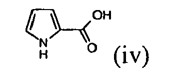 | EtOH | +++++ |
| Pyrazole 5- carboxylic | 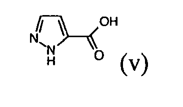 | H₂0 | - |
| **Pyrazole 4-chloro 5-carboxylic** | 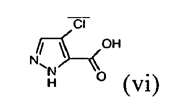 | H₂0 | ++ |
| **Pyrazole 4-bromo 5-carboxylic** | 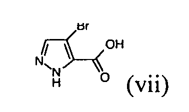 | H₂0 | ++ |
| Pyrazole 3-methyl 5 carboxylic | 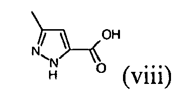 | H₂0 | - |
| Pyrazole 3-5 dicarboxylic | 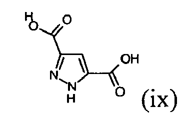 | H₂0 | - |
| Pyrazole 4-nitro 5 carboxylic | 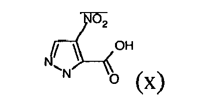 | H₂0 | - |
| 1-H Imidazole 2-carboxylic | 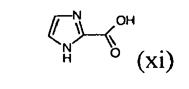 | H₂0 | - |
| 1-H Imidazole 4,5 dicarboxylic | 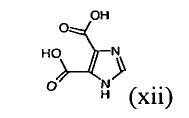 | H₂0 | - |
| Pyridine 2,3-dicarboxylic | 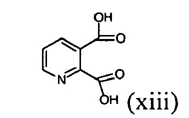 | H₂0 | - |
| Picolinic | 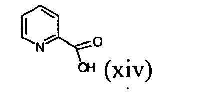 | H₂0 | - |
| Oxazole 4methyl 5 carboxylic acid | 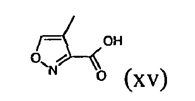 | H₂0 | - |
| Oxazole 4 isopropyl 5 carboxylic acid | 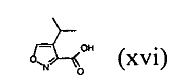 | H₂0 | - |
| Oxazole 4 ethyl 5 carboxylic acid | 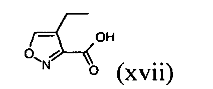 | H₂0 | - |
| Pyrazole 3 -methyl, 4-(Chloro), (Bromo), 5-carboxylic | 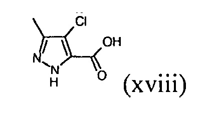 | H₂0 | - |
| Pyrazole 4-ethyl 3-carboxylic | 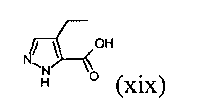 | H₂0 | - |
| Pyrazole 4-isopropyl 5-carboxylic | 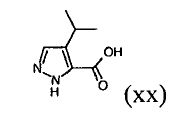 | H₂0 | - |
| Pyrazole 4-metapyridinyl 5 carboxylic acid | 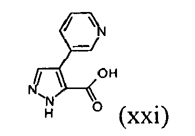 | H₂0 | - |

| | | | |
|---|---|---|---|
| * As determined by polarimetry and DAAOx methodologies (see Example 1. Material and Methods) | | | |

### 2.2 Two new TcPRAC inhibitors are revealed by conformation studies based on the flexibility of the enzyme pocket upon PYC binding.

Studies were pursued concerning the structure of the synthesized PYC analogues and their biochemical properties and comparisons were made for instance between PYC and the two most active ligands *versus* the inactive compounds in the context of the *Tc*PRAC catalytic site.

Molecular dynamic-based methods were applied to *Tc*PRAC-ligand docking by generating a number of conformation models compatible with the inventors experimental data, so as to gain further insights into energy transition states that could be further stabilized by ligand binding. The use of an ensemble of protein conformations as "target" for docking was important to take into account the *Tc*PRAC flexibility observed upon ligand binding (see Material and Methods and Figure 2).

Several models were then generated using the program FlexX 2.0.3 and 4 of these were used for the virtual screening of 31000 compounds of the Chimiohèque Nationale and 63000 compounds of ChemDiv chemical libraries (www.chemdiv.com; www.chimiotheque-nationale.enscm.fr) . While two of those models allowed the docking of small ligands, two other allowed the selectivity of ligands that were larger than PYC.

Two ligands identified using this molecular dynamic method were the acrylates - *(E)*-4-oxopent-2-enoic acid (OxoPA, C₅H₆O₃, MW 114,10) and its derivative *(E)*-5-bromo-4-oxopent-2-enoic acid (Br-OxoPA, C₅H₅O₃Br, MW 193,1) (see Figure 3 for their structures, provided as Formula (ii) and (iii) respectively).

Those compounds were shown to be 2 and 3 times better inhibitors of *Tc*PRAC than PYC. The data was obtained by the D- amino oxidase test and were confirmed by polarimetry (see Tables 2A and 2B). Preliminary results showed that the Kᵢ of one of these molecules (OxoPA) is lower (2,16 µM) than the one of PYC (8,46 µM) (see Figure 4).

**Table 2A: Two new inhibitors of TcPRAC presenting more affinity than PYC (DAAOx test).**

| **Ligand** | **Ligand Concentration** | **Formed D-Proline** |
|---|---|---|
| PYC | 10 µM | 2.4 mM (IC50) |
| OxoPA | 10 µM | < 0,3 mM |
| Br-OxoPA | 10 µM | < 0,3 mM |

The values presented in Table 2A correspond to racemization reactions performed in the absence or in the presence of 10 µM concentration of the different ligands. Note that approximately 10 µM PYC is necessary to inhibit 50% of the racemization of 10 mM L-proline (i.e. 2.5 mM formed D-Proline = IC50) by the DAAO test. Briefly, 22.4 mg/ml of active *Tc*PRAC was used in the racemization assays of 10 mM L-proline substrate and the concentration of formed D-proline was determined by the DAAOx test, as decribed in the Material and Methods.

**Table 2B: Two new inhibitors of TcPRAC presenting more affinity than PYC (polarimetry)**

| **Ligand** | **Ligand concentration** | **Racemization Inhibition** |
|---|---|---|
| PYC | 10.0 µM | 56.9% |
| OxoPA | 3.0 µM | 50.0 % |
| Br-OxoPA | 2.5 µM | 50.0 % |
| Br-OxoPA | 10.0 µm | 81.3% |

The values presented in Table 2B correspond to racemization reactions performed in the absence or in the presence of the different inhibitors. Note that 6 µM PYC is necessary to inhibit 100 % of the racemization of 10-20 mM L-proline [15]. Briefly, L-Proline (20 mM) were incubated for 30 minutes at 37°C in test tubes with or without 0.25 µM *Tc*PRAC in 0.2 M sodium acetate pH 6. Reactions were stopped by heating at 65° C for 10 minutes. Reaction volumes were analyzed for D-Proline formation, by measuring optical rotations in a polarimeter (365nm).

Finally both the compounds OxoPA and Br-OxoPA were tested at a concentration of 10 µM for toxicity of Vero cells for 48h. Both compounds are not toxic as evaluated by Trypan Blue exclusion (not shown).

### 2.3 The mammalian cell invasion by the parasite is hampered by new inhibitors of TcPRAC

To further study the effects of the new proline racemase inhibitors, cultures of Vero cells were set up in the presence or absence of different concentrations of the compounds and compared to earlier data obtained with PYC. The inventors previous results showed that when increasing PYC concentrations (10µM, 100 µM, 1000 µM) were added concomitantly with interaction of trypomastigotes and host cells *in vitro,* a clear dose-dependent effect was noticed with less infected host cells and lower mean numbers of parasite per infected host cell.

As can be seen in Table 3A, higher lower doses of OxoPA or Br-OxoPA (100 and 30 times, respectively) are necessary to obtain the same endocytic index (EI) observed with culture cells treated with PYC. For instance, whereas control samples treated only with comparable amounts of DMSO did not show particular alterations, equivalent EI values are observed for 1000 µM PYC and 10 µM OxoPA or 30 µM of Br-OxoPA. The data showed equally a dose dependent effect of the treatments with OxoPA and Br-OxoPA demonstrating a considerable decrease in both the percentage of host cells infected and the mean number of parasites found per cell as compared with the untreated sample (Tables 3B and 3C).

**Table 3A : Endocytic index**

| | **0** | **3 µM** | **10 µM** | **30 µM** | **100 µM** | **1000 µM** |
|---|---|---|---|---|---|---|
| *PYC** | *45* | *nd* | *44* | *nd* | *30* | *25* |
| OxoPA | 57 | 41,8 | 22,96 | 21,5 | nd | nd |
| Br-OxoPA | 57 | 77,7 | 42,3 | 28,7 | nd | nd |

**Table 3B : % Infected cells**

| | **0** | **3 µM** | **10 µM** | **30 µM** | **100 µM** | **1000 µM** |
|---|---|---|---|---|---|---|
| *PYC** | *90,0* | *nd* | *75, 0* | *nd* | *45,0* | *25, 0* |
| OxoPA | 38,6 | 26,8 | 16,6 | 13,9 | nd | nd |
| Br-OxoPA | 38,6 | 37,7 | 23,5 | 13,5 | nd | nd |

**Table 3C : Mean number of parasites/ cell**

| | **0** | **3 µM** | **10 µM** | **30 µM** | **100 µM** | **1000 µM** |
|---|---|---|---|---|---|---|
| *PYC** | *2* | *nd* | *1,7* | *nd* | *1,5* | *1,0* |
| OxoPA | 1,5 | 1,6 | 1,4 | 1,5 | nd | nd |
| Br-OxoPA | 1,5 | 2,0 | 1,8 | 1,6 | nd | nd |

Vero cells (from green monkey kidney) were seeded at a density of 15x10⁶ cells per 25cm² culture flasks or at 5x10⁴ cells/well into LabTek slides. The cultures were maintained in RPMI 1640 medium supplemented with 5% fetal bovine serum and kept at 37°C in an atmosphere of 5% CO². Cell culture-derived trypomastigotes from *T.cruzi* CL Brener (clone F11-F5) were isolated from the supernatant of Vero cells, which had been previously infected with bloodstream trypomastigotes. LabTek wells were infected for 17h at 37°C with cultured trypomastigotes at a 10:1 parasite/cell ratio in the presence or in the absence of increasing doses of a freshly made dilutions of 3-30 µM of OxoPA or Br-OxoPA molecules. Cultures were washed, fixed and stained with Giemsa. *At this stage, for comparison, results obtained from cultures seeded with or without 10-1000 µM specific proline racemase inhibitor pyrrole-2-carboxylic acid (PYC) are shown and were obtained from our previous work [19]. Endocytic index was calculated as the % of infected cells X the mean number of intracellular parasites per infected cell. Nd = Not done

### 2.4 The new inhibitors of TcPRAC affect parasite multiplication inside the mammalian cells

Additional studies were performed to verify whether *Tc*PRAC inhibition by the new compounds would influence the intracellular fate of the parasite. For this purpose, after infection for 17 hours, the host cells were washed and different ligand concentrations (3µM, 10 µM, 30 µM) were added to the medium and the infection was followed for up to 48 hours. As can be observed in Tables 4A, B and C, after 48 hours of culture treatments, there was a dose-dependent decrease in the number of parasites/ 100 cells, as well as in the percentage of infected cells or yet in the mean number of parasites/cell. In conclusion, our experiments show that *Tc*PRAC inhibition may affect both cellular infection by *T. cruzi* as well as its development in the tissue.

**Table 4A : Number of parasites/100 cells**

| | **0** | **3 µM** | **10 µM** | **30 µM** |
|---|---|---|---|---|
| OxoPA | 153,15 | 121,3 | 129,4 | 56,1 |
| Br-OxoPA | 153,15 | 176,5 | 155,8 | 69,4 |

**Table 4B : % Infected Cells**

| | **0** | **3 µM** | **10 µM** | **30 µM** |
|---|---|---|---|---|
| OxoPA | 39,3 | 41,3 | 41,3 | 20,0 |
| Br-OxoPA | 39,3 | 55,3 | 49,8 | 31,0 |

**Table 4C : Mean number of parasites/cell**

| | **0** | **3 µM** | **10 µM** | **30 µM** |
|---|---|---|---|---|
| OxoPA | 3,9 | 2,9 | 3,0 | 2,8 |
| Br-OxoPA | 3,9 | 3,2 | 3,1 | 2,3 |

Vero cells (from green monkey kidney) were seeded at a density of 15x10⁶ cells per 25cm2 culture flasks or at 5x10⁴ cells/well into LabTek slides. The cultures were maintained in RPMI 1640 medium supplemented with 5% fetal bovine serum and kept at 37°C in an atmosphere of 5% CO2. Cell culture-derived trypomastigotes from *T.cruzi* CL Brener (clone F11-F5) were isolated from the supernatant of Vero cells, which had been previously infected with bloodstream trypomastigotes. LabTek wells were infected for 17h at 37°C with cultured trypomastigotes at a 10:1 parasite/cell ratio. All the wells were then washed to eliminate all extracellular parasites and new medium was added to the cultures containing or not increasing concentrations of freshly made dilutions of 3-30 µM of OxoPA or Br-OxoPA molecules. Cultures were washed after 48h, fixed and stained with Giemsa.

### 2.5 Conclusion:

The data presented herein substantiate previous findings showing that proline racemase plays a role in both infectivity and differentiation of *T. cruzi* and further support its use as a critical target to the development of a chemotherapy against Chagas disease. In conclusion, these experiments show that *Tc*PRAC inhibition by OxoPA or Br-OxoPA affects both cellular infection by *T*. *cruzi* as well as its development in the tissue support the claim that those chemical compounds may be further developed as leads to new therapies against chronic phases of the disease. The inventors have recently shown that important pathogens of the same family of *T. cruzi* [29] and also nosocomial bacteria produce functional proline racemases [30], our data strongly suggest that those chemical may also apply to the development of new therapeutic agents against those and other pathologies where proline racemases are important.

### Example 3. An integrated approach to unravel short and low energy path for complex transitions in proteins and biopolymers.

### 3.1 Introduction:

Although, allostery and molecular motions have been known for a long time, their description at the atomic level remains a challenge. The description of the thermodynamic characteristics would be a collection of possible paths through a wide variety of intermediate structure sequences and probability distributions. However, for many applications and simplicity, it is also useful to describe those paths with a more deterministic description.

This simplification of the description can be paralleled to the implications of the Levinthal paradox which proposes a funnel-like path for folding. That is a proteins 3D structure or final confirmation is achieved without the need for the linear peptide sequence to adopt each possible 3D confirmation before arriving at the correct one, but instead the peptide attempts to adopt a much more limited set of structure which inevitably leads to the correct one.

A "backbone" of the transition could serve to describe it at a certain level. Another aspect is that a simplified view with a "backbone" description of transition yields detailed, atomistic information on plausible intermediate states which can serve in the same way as a crystallographic structure for analysis and for use in prediction tools.

As a consequence, molecular motions such as local or global transconformations and allostery in a wide sense can be used in practice as a new class of therapeutic target as they are often linked to biological function. Examples of applications are described elsewhere.

The progress in structural biology has provided data showing the existence of these motions in a wide variety of biological processes in the form of crystallographic or NMR structures for various states of biological systems such as signal transduction, proton pumps, allosteric enzymes, mechanical motions of muscles and flagella. However, the actual 3D description at atomic level remains limited to the use of those experimental "end" state structures or to structures that can be modelled from those with classical methods.

For analysis and other applications like rational, structure based, drug design, the use of reliable intermediate models is of great interest for understanding and extending these possible applications.

Molecular Dynamics, (MD), is a powerful method, but to date is confined to simulations over nano to micro seconds time scales. Such time frames are not suited to the study of transitions involving barrier crossing over longer time scales. Computational methods have been developed to study large conformational changes by targeted as well as steered molecular dynamics with an additional constant or evolving biasing potential, driving to a target conformation. Normal Mode-following algorithms can be used to find transition states by following the energy landscape to saddle points [33].

Various low energy path methods have been developed in which the path for a conformational change is quantified with a series of images of the molecule spaced along the path. The images at the endpoints are fixed in space. Each image between is connected to the previous and next image. The path represents the trajectory that a molecule follows through the conformational change and this path can be derived independently of the timescale for the conformational change.

Among those methods, the Nudged Elastic Band (NEB) uses springs to connect intermediate structures [34, 35, 36]. The Conjugate Peak Refinement (CPR) builds incrementally and indirectly the second derivative matrix to identify saddle points [37]. Other methods have also been described [38].

However, those calculations remain a challenge [39], as the existing methods are very good at refining path towards locally low energies, but are easily trapped for complex transitions. As a consequence long or high energy path are produced for systems involving transitions more complex than simple local conformer shifts. If long path are created to overcome local barriers, the course of the refinement becomes chaotic and dependent on history.

### 3.2 Methods and Definitions.

### 3.2.1 Path

In the course of the description of the path calculations, the inventors call a path a series of structures linking two predefined end structures of a system in the context of an energy function in such a way that any intermediate structure of the series or any linear interpolation of two consecutive structures of that series has an energy below a given threshold. The structures can be defined by the unique set of cartesian coordinates for each component considered in the system in such a way that the energy function is defined. Typically, it can be understood as the coordinates of the atomic nuclei as it can be found in a PDB coordinate file.

Therefore, in a path p composed on Np structures, for each index, i, for i = 1 to Np, the structure is given by a vector Xp,i. Xp,1 and Xp,Np shall be the vectors defining the end structures for which a connecting path is being looked for. The energy E(X) is defined for any configuration or structure specified by X. Intermediate structures can be either one of Xp,i for i in [1,..,Np] or given by Xp,i,1= (1-1)*Xp,i+1*Xp,(i+1) for i in [1,..,Np-1] and 1, a real number in [0,1]. A path can also be noted Xp,i(in [1,Np]) or {Xp,1;Xp,2;...;Xp,Np}. The path {Xp,1;Xp,Np} with Np=2 is the simplest path; in none-trivial situations, the energy threshold as defined below is expected to correspond to a bad quality criterion, as defined below.

By extension, it can be applied on any space or vectorial space upon which interpolations, a metric and a score function can be defined.

### 3.2.2 Probing the energy or score function

To test that any intermediate or interpolated structure is below the given threshold, any one dimensional maximum research algorithm can be used on each interval of the path (search Max(E(Xp,i,1)) on 1 for each i(in [1,Np-1])). Xp,1 and Xp,Np shall be written Xp,1,0 and Xp,Np,0 in this notation.

### 3.2.3 Metrics

The lengths of the paths can be evaluated according to measures. The distance covered, d, is the distance between Xp,1 et Xp,Np according to the metrics of the considered space. The Euclidian distance, or RMS, or d(Xp,1;Xp,Np) can be used: d=sqrt((Xp,1-Xp,Np)*(Xp,1-Xp,Np)/Ndeg), Where * denotes the scalar product between vectors and Ndeg is the relevant number of degrees of freedom (the number of atoms or 3 times the number of atoms, depending on the convention, and as this approach can be applied to any vectorial path search). The curvilinear length, Cl between any pairs of intermediate structures Xp,i,li; Xp,j,1j for (i<j or (i=j and li<lj))and i and j both in [1,Np] and li and lj both in [0,1], is the sum of distances bridging the those structures along the path: Cl(Xp,i,li; Xpj,lj) is d(Xp,i,li;Xp,j,lj) if j=i, or else, d(Xp,i,li;Xp,i+1)+sum(d(Xp,k;Xp,(k+1)), for k in i+1,j-1)+d(Xp,j,0;Xp,j+li).

### 3.2.4 Quality criteria

Various criteria can be used to evaluate the quality of a path. The energy and more specifically the maximum energy along the path can be one of these criteria. In a physical sense, the lower that maximum energy, the better is the quality. The curvilinear length and the number of structures composing the path (Np, above) are also criteria which indicates that the amount of unnecessary swerving is as low as possible.

### 3.2.5 By-pass

A by-pass is a path connecting Xp,i and Xp,j which can be different from Xp,k(in [i,j]) and can be noted Xb,i(in [1,Nb]), Xb,1=Xp,i and Xb,Nb=Xp,j. Useful by-passes are either composed of less structures Nb<j-i+1, shorter in length (CurvLength(Xb,k(in[1,Nb]))< CurvLength(Xp,k(in [i,j]))), lower in energy (Max(E(Xb,k(in [1,Nb]),1))< Max(E(Xp,k(in [i,j]),1))) or any combination of these properties.

### 3.2.6 Procedures

A procedure on the path is an algorithm which is provided with a path p: Xp,i(in [1,..,Np]) and which returns a new path p': Xp',i(in [1,..,Np']), such that Xp,1=Xp',1 and Xp,Np=Xp',Np'. Any method which generates new configurations can be used. On molecular systems, Molecular dynamics, Brownian dynamics, Monte Carlo methods, other path calculation methods, geometrical algorithms can be considered among others with or without additional constrains functions in addition to the score function.

### 3.3.1 Approach

The approach that is defined herein is composed of a series of procedures and provides plausible paths in the sense that, for a given energy function, the barriers are below levels that can be reached because they are relevantly populated in the thermodynamic sense at physiological condition.

### 3.3.1.1- Reduction procedure "Backwards"

The reduction procedure is devised in the following manner: for a given threshold (Tr), the path is iteratively truncated. At each step k, the sub-path Xp,i(in [nk,Np]) is considered. n1 is 1. In this sub-path, the highest index nk' for which {Xp,nk;Xp,nk'} is a path respecting the Threshold Tr shall be the next index n(k+1). The procedure stops when k'=Np, at step number Nr. The path p' produced by the reduction procedure has Np'=Nr points and is given by Xp',i(in [1,Np'])=Xp,nk(in [1,Nr]).

### 3.3.1.2 Wider by-passes.

In a path Xp,k(in [1,Np]), the maximum energy, located between Xp,i and Xp,j, j>i, can be overcome with by-passes. By-passes can be of the form Xb,k(in [1,Nb]) with Xb,1=Xp,i and Xb,Nb=Xp,j. Alternatively, by-passes can be searched to connect any pair of bracketing points Xp,i';Xp,j' for i'<=i and j'>=j. Any procedure as defined in the "Procedures" section can be applied on paths {Xp,i';Xp,j'} for different combinations of i',j' to calculate useful by-passes candidates as defined in "By-pass". The same approach can be used to find shorter straddling sub-paths, Xp,i';Xp,j' for i'<j and j>i, but will necessitate reassembly of partial by-passes to cover the Xp,i;Xp,j interval. Typically partial by-passes covering Xp,i';Xp,j' and Xp,i";Xp,j" and such that i'<=i,j">=j, andj'>=i" given by Xb1,i(in [1,Nb1]) and Xb2,i(in [1,Nb2]) can be assembled by simple concatenation: {Xb1,i(in [1,Nb1]);Xb2,i(in [1,Nb2])} and subject to procedure described in the "Reduction procedure "Backwards"" section.

### 3.3.1.3 Heuristics to search for by-passes: systematic search.

The pairs of indexes i',j' as defined in section "Wider by-passes", can be tested successively according to the following schemes. For increasing values of m starting from 0 and all indices n (in [0,m]), i'=i-m+n and j'=j+n (j'-i'=j-i+m). The search is stopped when a given criteria as defined in "Quality criteria" is reached. A maximum value of m, mmax can be set to limit the search to a reasonable amount of computational power. Note that this heuristic can be computationally implemented in a "trivial parallelisation" way (series of independent computer runs).

### 3.3.1.4 Heuristics to search for by-passes: priority search

The number of possible pairs of structures that can be used to search by-passes is Np*(Np+1)/2, (more than 5000 for Np=100 or half a million for Np=1000, etc...). The procedure used to build by-passes may provide useful ones, as defined in the "By-pass" section, for a minority of the cases. Some pairs of structures, i,j, based on selection criteria are more prone to provide such useful by-passes. Such criteria where found to be the distance (d(Xp,i;Xp,j)), the number of intervals (j-i), the ratio of distance to the curvilinear length (d(Xp,i;Xp,j)/Cl(Xp,i;Xp,j)), but also the systematic search for a by-pass for a series of energy maximum for m in [0,mmax] as defined in the "Heuristics to search for by-passes: systematic search" section. Those criteria are used to build a list of pairs ik,jk k(in [1,N1]) for which by-passes ending at Xp,ik;Xp,jk are searched for. The criteria can be adjusted so that N1 corresponds to a reasonable amount of computational effort (a few hundred to a few thousand at current time). The recipes can be: pairs i,j such that j-i>Nmin, d(Xp,i;Xp,j))<dmax and d(Xp,i;Xp,j)/Cl(Xp,i;Xp,j)<rmax or which allow the by-pass of listed maxima with m < mmax.

### 3.3.1.5 Selection of by-passes.

By-pass, indexed k between Xp,ik;Xp,jk are noted Xbk,i(in [1,Nbk]). By-passes were selected on the basis of criteria defined in the "By-pass" section. Nbk<jk-ik and Max(E(Xbk,i(in [1,Nbk]),1(in [0,1]))) lower than Max(E(Xp,k(in [i,j]),1(in [0,1]))) were used extensively.

### 3.3.1.6 Assembly of by-passes.

The assembly of By-passes Xbk,i(in [1,Nbk]) for k in [1,Nk] to reconstitute a new path is a combinatorial problem. For one series of Nc k values, kc(c in [1,Nc]) of compatible by-passes Xbkc,i(in [1,Nbkc]), Xp,i(in [1,Np]) is replaced by {Xp,i(in [1,ik1-1]);Xbk1,i(in [1,Nbk1]; Xp,i(in [jk1+1,ik2-1]);Xbk2,i(in [1,Nbk2];.....;Xp,i(in [jkNkc,Np)}. The notation can be different for combinations of path and/or by-passes which have identical ends. Compatible by-passes can be bypasses for which jk1<=ik2 or inversely jk2<=ik1, but it can more generally be a combination of by-passes which yields a path for a relevant threshold after the application of a procedure as defined in the "Procedure" section. A tree search was implemented to build the series of compatible by-passes, kc,s(c in [1,Nc];s in [1,Ns]). In the construction of that tree, by-passes k and k' were considered compatible if jk<=ik' or inversely if jk'<=ik. The assembly of the path/by-passes as described above was implemented for each series of kc,s(c in [1,Nc];s in [1,Ns]) and was subjected to further procedure as defined in the "Procedures" section to yield new paths Xps,i(in i,Nps). The number of such series of compatible by-passes, Ns, is increasing rapidly with the number of compatible by-passes, Ncomp (Ns is up to 2 to the power of N_{comp} (2^{Ncomp})). It may then be useful to iteratively make sub-trees to limit the combinatorial in the search for the best combinations. However, to fully evaluate the benefit of a combination of by-passes which are confluent or close to each others, it is important to evaluate those combinations directly in a tree search. The meaning of confluent can be understood as jk1=ik2 or jk1=ik2+1, while that of close depends on the characteristics of the procedure that will be applied subsequently which results can depend on a few flanking structures. The best combinations of by-passes can be selected based directly on the energy, number of intermediate structures and curvilinear length of the path assembled with them or after application of a procedure on the latter.

### 3.4 Application for drug design.

### 3.4.1- Catalytic/motions/allosteric. Positive/negative selection

Applications can use intermediate states as structural targets in a rational drug design process. For that, various strategies can be applied. Intermediate states can be used in various ways to block or activate a biological function:
- It can be used to block a structural transition by stabilization of an intermediate state and thus, structurally sequestering conformation out of the end, functionally active, conformations;
- It can be used to destabilize an intermediate or end structure to affect the transition.
- It can be used to discriminate structural targeting according to the evolution of a target site along the transition.

Those modes of action are not mutually exclusive.

Those strategies and intermediate structures can be, *a priori,* or *a posteriori,* validated or confirmed by physical-chemistry data on ligand binding and comparison with the predicted fitness of those ligands on the intermediate structure.

### 3.5 - Building of a transition path between open and closed forms of TcPRAC

A framework approach was used to calculate plausible low energy and connected intermediate states in *Tc*PRAC conformational transitions. This is defined by a series of intermediate structures, which are all below an energetic threshold and for which any linear interpolation between any pairs of consecutive structures are also below that threshold.
The *Tc*PRAC hemi-bounded structure (1 W62) is asymmetric (chain A closed on PYC, chain B opened). The symmetric structure was built by axial rotation and orientation (chain A, B, opened, closed resp.) with CHARMM [48]. The solvent was modeled implicitly with the analytical continuum electrostatics (ACE2) potential [49] in order to prevent artifacts on modeled cavities. The dielectric constant was 1 inside the protein and 80 outside. Internal coordinates and Cartesian coordinates of the two extreme structures were compared to remove unnecessary symmetric side-chain flips.

An initial estimate for the transition path was built by recording a Molecular Dynamics of 200,000 steps by sampling every 100^{th} step. It started from one structure and was drawn to the other structure with a linear constrain of 0.5 kcal/mol/Å/atom. A Langevin thermostat at 300K with a friction coefficient of 100 ps⁻¹ removed the excess heat.

The refinement of the path was performed with two aims: a) maintain the number of framework and minimize the length, b) build low energy intermediate states. This was performed with various procedures which can be combined to: (i) remove intermediate structures if any linear interpolation between remaining consecutive structures still are below an energy threshold, "backward reduction". (ii) replace sub-paths by recalculated sub-path candidates, these replacement sub-paths can be of increasing width. (iii) systematic search for sub-path candidates as defined in (ii), or, (iv) priority search for such sub-paths with criteria allowing an *a priori* selection of more potent candidates. (v) selection of sub-paths according to quality criteria, and (vi) assembly of sub-paths respecting the integrity of the transition with a tree-type approach and some heuristic to contain combinatorial expansion.

The resulting path was thus a series of intermediate structures bridging the two symmetric states of the enzyme without energy barriers.
Step (ii) could be performed by molecular mechanics or with a Conjugate Peak Refinement (CPR) algorithm [50]. The energy reduction threshold, (i), was -34000 kcal/mol. The quality criteria (v) were: a) lower highest energy than that of the whole path b) less intermediate points than the replaced sub-path. If a) is violated by the original sub-path and no candidates fullfill a)&b), take sub-path candidates fulfilling a) uniquely.

Four intermediate structures were chosen to perform the virtual screening of about 700.000 molecules from two accessible chemical libraries: ChemDiv, Inc (San Diego, California, USA) and Chimiothèque Nationale (CN, CNRS, France). The following programs were used: FlexX 2.0.3 [47]. The selection of the ligand was based on the FlexX score, -20, -20, -25 and -33, for structures 1, 4, 10 and 49 of the transition path. To limit the number of molecule to test, a clustering of the molecules using a UPGMA classification with a 0.55 threshold was used and the best molecule of each cluster was kept.

### 3.6 - Building of intermediate conformations of TcPRAC to widen chemoinformatic search of inhibitors.

**The** catalytic site of *Tc*PRAC is small (volume of a proline) and thus virtual screenings previously performed with designed pharmacophores (not shown) were bound to yield small and metabolite like molecules. We built possible intermediates structures for the functional transition of *Tc*PRAC, bridging its unliganded/openned and its liganded/closed forms of the catalytic site [20]. This allowed widening the search to larger and chemically more diverse inhibitor candidates.

Molecular dynamics and molecular mechanics based methods were applied to *Tc*PRAC in the conformation found in 1W62 to infer plausible conformational intermediate states followed by the enzyme in its transition between the two symmetric hemi bound states (Figure 6a). The transition proved to be relatively simple as compared to other cases (e.g. [19] and the process converged after 3 full cycles of the procedure described in Material and Methods. The resulting path was composed of 49 structures with low energies (Figure 5). It is asymmetric and the length of the path was 4.78 Å to cover a distance of 2.62 Å between the to extreme conformations.

Known ligands, PYC, L-PRO, D-PRO, Pyrazole-4-chloro-5-carboxylic, Pyrazole-4-chloro-5-carboxylic, and Pyrazole-4-ethyl-3-carboxylic were docked in both catalytic sites for each of the 49 conformations with FlexX. The pocket corresponding to Chain A gave better results. Conformations 4 yielded good scores for some ligands that did not score well in conformation 1 (closed catalytic site for chain A in 1W62). Conformation 10 still yielded favorable scores and still had an organised hydrogen bond network in the catalytic site, which was not the case for following conformations. These two conformations together with the closed and opened crystallographic conformations were selected as "target" for the virtual screening.

### 3.7 - Virtual screening and ligand candidate selection.

The 4 models were used for the virtual screening of 31000 compounds of the Chimiothèque Nationale (CN) and 630000 compounds of ChemDiv chemical libraries. The first two conformations lead to the selection of small ligands, while conformation 10 and 49 promoted ligands that were larger than PYC. Noticeably, however, conformation 4 selected slightly larger ligands than conformation 1. Following the procedure explained in material and methods, 113 compounds were ordered from the ChemDiv and 37 from the CN and respectively XX and 26 compounds finally arrived.

Among these compounds, two ligands from the CN are the acrylates - *(E)*-4-oxopent-2-enoic acid (OxoPA, C₅H₆O₃, MW 114,10) and its derivative *(E)*-5-bromo-4-oxopent-2-enoic acid (Br-OxoPA, C₅H₅O₃Br, MW 193,1) (Figure 3).

### References

1. O'Connell D (2007) Neglected Diseases. Nature 449: 157.
2. Aznar C, La Ruche G, Laventure S, Carme B, Liegeard P, Hontebeyrie M (2004) Seroprevalence of Trypanosoma cruzi infection in French Guiana. Mem Inst Oswaldo Cruz 99: 805-808.
3. Commission Européenne (2006) Portant application de la directive 2004/23/CE du Parlement européen et du Conseil concernant certaines exigences techniques relatives au don, à l'obtention et au contrôle de tissus et de cellules d'origine humaine. Journal officiel de 1'Union européenne. 9 février 2006, pp. L38/40-L38/52.
4. CDCP (2007) Blood Donor Screening for Chagas Disease - United States, 2006-2007. 56(7): 141-143 p.
5. CDCP (2006) Chagas Disease After Organ Transplantation - Los Angeles, California, 2006. 55(29): 798-200 p.
6. Garraud O, Andreu G, Elghouzzi MH, Laperche S, Lefrere JJ (2007) Measures to prevent transfusion-associated protozoal infections in non-endemic countries. Travel Med Infect Dis 5: 110-112.
7. Chamond N, Coatnoan N, Minoprio P (2002) Immunotherapy of Trypanosoma cruzi infections. Curr Drug Targets Immune Endocr Metabol Disord 2: 247-254.
8. Coura JR, De Castro SL (2002) A Critical review on Chagas disease chemotherapy. Mem Inst Oswaldo Cruz 97: 3-24.
9. Docampo R (1990) Sensitivity of parasites to free radical damage by antiparasitic drugs. Chem Biol Interact 73: 1-27.
10. Maya JD, Bollo S, Nunez-Vergara LJ, Squella JA, Repetto Y, Morello A, Perie J, Chauviere G (2003) Trypanosoma cruzi : effect and mode of action of nitroimidazole and nitrofuran derivatives. Biochem Pharmacol 65: 999-1006.
11. Cancado JR (1999) Criteria of Chagas disease cure. Mem Inst Oswaldo Cruz 94 Suppl 1: 331-335.
12. Cançado JR (2002) long term evaluation of etiological treatment of chagas disease with benznidazole. Rev Inst Med trop S Paulo 44: 29-37.
13. WHO (2006) Chagas disease (http://www.who.int/ctc/chagas/disease.htm).
14. Morel C, Carvalheiro J, Romero C, Costa E, Buss P (2007) Neglected diseases : the road to recovery. Nature 449: 180-182.
15. Chamond N, Gregoire C, Coatnoan N, Rougeot C, Freitas-Junior LH, da Silveira JF, Degrave WM, Minoprio P (2003) Biochemical characterization of proline racemases from the human protozoan parasite Trypanosoma cruzi and definition of putative protein signatures. J Biol Chem 278: 15484-15494.
16. Chamond N, Coatnoan N, Goytia M, Barale J-C, Cosson A, Degrave WM, Minoprio P (2005) Trypanosoma cruzi proline racemases are involved in parasite differentiation and host cell invasion. Mol Microbiol 58: 46-60.
17. Reina-San-Martin B, Degrave W, Rougeot C, Cosson A, Chamond N, Cordeiro-Da-Silva A, Arala-Chaves M, Coutinho A, Minoprio P (2000) A B-cell mitogen from a pathogenic trypanosome is a eukaryotic proline racemase. Nat Med 6: 890-897.
18. Keenan MV, Alworth WL (1974) The inhibition of proline racemase by a transition state analogue: delta-1-pyrroline-2-carboxylate. Biochem Biophys Res Commun 57: 500-504.
19. A. Blondel and J. P. Renaud and S. Fischer and D. Moras and M. Karplus, Retinoic acid receptor: a simulation analysis of retinoic acid binding and the resulting conformational changes, (1999) J Mol Biol, 291, 101-115.).
20. Buschiazzo A, Goytia M, Schaeffer F, Degrave W, Shepard W, Gregoire C, Chamond N, Cosson A, Berneman A, Coatnoan N, Alzari PM, Minoprio P (2006) Crystal structure, catalytic mechanism, and mitogenic properties of Trypanosoma cruzi proline racemase. Proc Natl Acad Sci U S A 103: 1705-1710.
21. Gouault N, Pinel B, Cupif JF, Depince A, Martin-Chouly CA, Belleguic C, David M (2004) Synthesis and potential anti-inflammatory activity of some tetrahydrophthalazinones. J Enzyme Inhib Med Chem 19: 475-480.
22. Manaev YA, Andreeva MA, Perevalov VP, Stepanov BI, Dubrovskaya VA, Seraya VI (1982) Syntheses from dimethylpyrazole V. Nitration of-3-and-5-carboxylic acids. JGenChemUSSR (EnglTransl) 52: 2291-2296 See Chem. Abstr. 2298:71993.
23. Fatin-Rouge N, Toth E, Perret D, Backer RH, Merbach AE, Bunzli J-CG (2000) Lanthanide podates with programmed intermolecular interactions: luminescence enhancement through association with cyclodextrins and unusually large relaxivity of the gadolinium self-aggregates. J Am Chem Soc 122: 10810-10820.
24. Reina-San-Martin B, Cosson A, Minoprio P (2000) Lymphocyte polyclonal activation: a pitfall for vaccine design against infectious agents. Parasitol Today 16: 62-67.
25. Berneman A, Alves-Ferreira M, Coatnoan N, Chamond N, Minoprio P (2009) High throughput D-amino acid oxidase colorimetric method for determination of D-amino acids. Application for amino acid racemases. submitted.
26. de Souza EM, Araujo-Jorge TC, Bailly C, Lansiaux A, Batista MM, Oliveira GM, Soeiro MN (2003) Host and parasite apoptosis following Trypanosoma cruzi infection in in vitro and in vivo models. Cell Tissue Res 314: 223-235.
27. da Silva CF, Batista MM, Batista Dda G, de Souza EM, da Silva PB, de Oliveira GM, Meuser AS, Shareef AR, Boykin DW, Soeiro Mde N (2008) In vitro and in vivo studies of the trypanocidal activity of a diarylthiophene diamidine against Trypanosoma cruzi. Antimicrob Agents Chemother 52: 3307-3314.
28. Corsano S, Capito L, Bonamico M (1958) Research on the structure of glutinic acid. I. Heterocyclic derivatives of the allenic acids. Ann Chim (Rome) 48: 140-155.
29. Chamond N, Cosson A, Coatnoan N, Minoprio P (2009) Proline racemases are conserved mitogens: Characterization of a Trypanosoma vivax proline racemase. Mol Biochem Parasitol 165: 170-179.
30. Goytia M, Chamond N, Cosson A, Coatnoan N, Hermant D, Berneman A, Minoprio P (2007) Molecular and structural discrimination of proline racemase and hydroxyproline-2-epimerase from nosocomial and bacterial pathogens. PLoS ONE 2: e885.
31. Buckner FS, Wilson AJ, White TC, Van Voorhis WC (December 1998). "Induction of resistance to azole drugs in Trypanosoma cruzi". Antimicrob Agents Chemother 42 (12): 3245-50.
32. Lauria-Pires L, Braga MS, Vexenat AC, et al. (2000). "Progressive chronic Chagas heart disease ten years after treatment with anti-Trypanosoma cruzi nitroderivatives". Am J Trop Med Hyg 63 (3-4): 111-8.
33. Henkelman G. & Jonsson, H., J. Chem. Phys. (1999) 111, 7010.
34. Elber R. & Karplus M. Chem. Phys. Letters, (1987) 139, 375-380.
35. Mills G. & Jonsson H. Phys. Rev. Letters, (1994) 72, 1124-1127.
36. Jonsson H., Mills G. & Jacobsen K. W. (Berne B. J., Ciccoti G. & Coker, D. F., eds, 1998), pp. 385-404 Wold Scientific, Singapore.
37. Fischer S. & Karplus M. Chem. Phys. Letters, (1992) 194, 252-261.
38. Ayala P. Y. & Schlegel H. B. J. Chem. Phys. (1997) 107, 375-384.
39. PG. Bolhuis, D. Chandler, C. Dellago & PL. Geissler, Ann. Rev. of Physical Chemistry (2002) 53: 291-318.
40. S.M. Berge, L.D. Bighley, D.C. Monkhouse, J. Pharm. Sci. 1977, 66, 1-19.
41. L.D. Bighley, S.M. Berge, D.C. Monkhouse, in "Encyclopedia of Pharmaceutical Technology'. Eds. J. Swarbrick and J.C. Boylan, Vol. 13, Marcel Dekker, Inc., New York, Basel, Hong Kong 1995, pp. 453-499.
42. Hanzlowsky A, Jelencic B, Recnik S, Svete J, Golobic A, Stanovnik B (2003) Regioselective synthesis of ethyl pyrazolecarboxylates from ethyl 3-[(dimethylamino)methylidene]pyruvate and diethyl 3-[(dimethylamino)methylidene]-2-oxosuccinate. Isolation of ethyl 4,5-dihydro-1-heteroaryl-5-hydroxy-1H-pyrazole-5-carboxylates as stable intermediates in the pyrazole ring formation. J Heterocyclic Chem 40: 487-498.
43. Stach H, Huggenberg W, Hesse M (1987) Synthesis of 2-hydroxy-3-methyl-2-hexen-4-olid. Helv Chim Acta 70: 369-374.
44. Auwers K, Cauer E (1929) Δ1 and Δ2-pyrazolines. Justus Liebigs Ann Chem 470: 284-312.
45. Alberti C, Zerbi G (1961) Sintesi di alchilpirazoli. Pharmaco Ed Sci 16: 527-539.).
46. Arndt F (1943) Diazomethane. Org Synth Coll Vol 2: 165-167.
47. Rarey, M., Kramer, B., Lengauer, T., Klebe, G. (1996). A Fast Flexible Docking Method using an Incremental Construction Algorithm. Journal of Molecular Biology, 261:470-489.
48. Bernard R. Brooks, Robert E. Bruccoleri, Barry D. Olafson, David J. States, S. Swaminathan, Martin Karplus, CHARMM: A program for macromolecular energy, minimization, and dynamics calculations (1983), Journal of Computational Chemistry, 4, 187-217).
49. Michael Schaefer and Martin Karplus, A Comprehensive Analytical Treatment of Continuum Electrostatics, (1996), J. Phys. Chem., 100(5), 1578-1599.).
50. Stefan Fischer and Martin Karplus, Simulation of activation free energies in molecular systems, (1996), J Chem Phys, 105, 1902-1921.)

SEQUENCE LISTING

<110>  Institut Pasteur

<120>  Inhibitors of the proline racemase of Trypanosoma cruzi

<130>  F226 - 171 EP

<160>  1

<170>  PatentIn version 3.3

<210>  1
<211>  423
<212>  PRT
<213>  Trypanosoma cruzi

<400>  1

## Claims

1. Use of a compound of the general Formula (i): 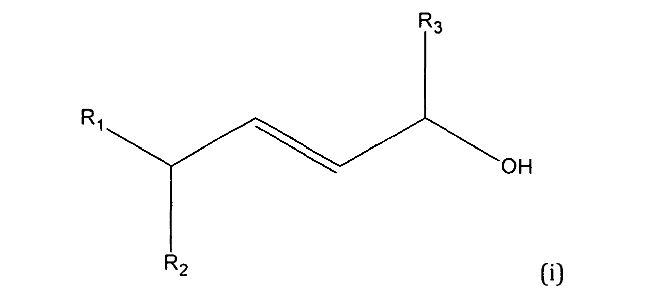 wherein R₁ is selected from the group consisting of: a substituted or unsubstituted C₁-C₁₀ alkyl group and a polar atom selected from the group consisting of: oxygen, nitrogen, sulfur or halogen atom; R₂ and R₃ are polar groups chosen from the group consisting of: =O, =NH, -NH₂, -OH, =S, -X₁ wherein X₁ is a halogen atom; and isomers thereof,
as a medicament.

2. The use of claim 1, wherein said alkyl group is selected from the group consisting of: methyl, ethyl, propyl, butyl, pentyl and isomers of these alkyl groups.

3. The use of claim 1 or 2, wherein said halogen atom is selected from the group consisting of: fluorine, chlorine, bromine, iodine.

4. The use according to any one of claims 1 to 3, wherein said compound is 4-oxopent-2-enoic acid (Formula ii) 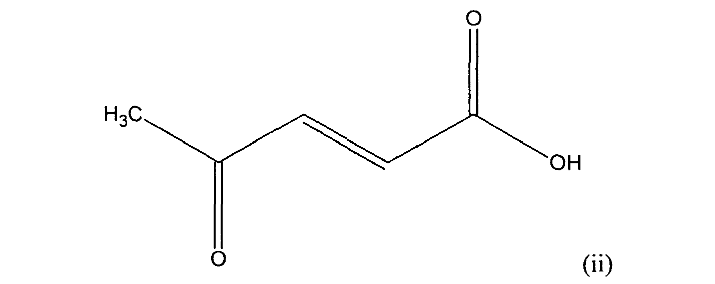 or a derivative thereof.

5. The use according to claim 4 wherein said derivative is *(E)*-5-bromo-4-oxopent-2-enoic acid (Formula (iii))

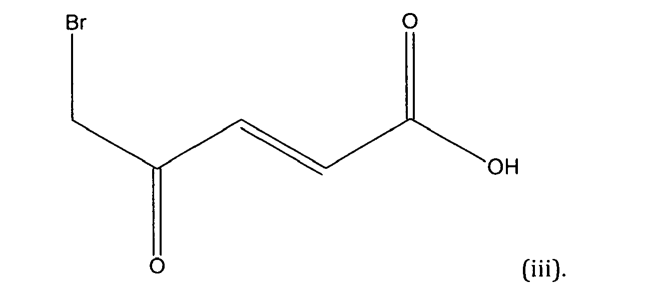

6. A medicament comprising at least one compound as claimed in any one of claims 1 to 5.

7. A pharmaceutical composition comprising at least one compound as claimed in any one of claims 1 to 5 and a pharmaceutically acceptable carrier.

8. The pharmaceutical composition according to claim 7, wherein said compound is in the form of a pharmaceutically acceptable salt or other pharmaceutically acceptable derivative of said compound.
